(19) Europäisches Patentamt
European Patent Office
Office européen des brevets

(11) Veröffentlichungsnummer: **0 294 686 B1**

(12) # EUROPÄISCHE PATENTSCHRIFT

(45) Veröffentlichungstag der Patentschrift: **09.12.92**

(51) Int. Cl.5: **C07C 283/00**

(21) Anmeldenummer: **88108662.3**

(22) Anmeldetag: **31.05.88**

(54) **Verfahren zur Herstellung von Iminokohlensäurediarylestern.**

(30) Priorität: **06.06.87 DE 3719015**

(43) Veröffentlichungstag der Anmeldung:
**14.12.88 Patentblatt 88/50**

(45) Bekanntmachung des Hinweises auf die Patenterteilung:
**09.12.92 Patentblatt 92/50**

(84) Benannte Vertragsstaaten:
**CH DE FR LI**

(56) Entgegenhaltungen:
**FR-A- 2 103 010**
**FR-A- 2 564 833**
**JP-B- 5 009 846**

**HOUBEN-WEYL: "Methoden der Organischen Chemie", Band E4, 1983, Seiten 561-569, Georg Thieme Verlag, Stuttgart, DE**

(73) Patentinhaber: **BASF Aktiengesellschaft**
**Carl-Bosch-Strasse 38**
**W-6700 Ludwigshafen(DE)**

(72) Erfinder: **Baus, Ulf, Dr.**
**Keltenweg 10**
**W-6915 Dossenheim(DE)**
Erfinder: **Reuther, Wolfgang, Dr.**
**Am Pferchelhang 16**
**W-6900 Heidelberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 294 686 B1

## Beschreibung

Die vorliegende Erfindung betrifft Verfahren zur Herstellung von Iminokohlensäurediarylestern sowie neue Iminokohlensäurediarylester.

Gemäß dem Stand der Technik, z.B. Houben-Weyl, Methoden der Organischen Chemie, Bd. E4, Seite 544 ff (1983) können N-substituierte Iminokohlensäurediarylester, die als wertvolle Ausgangs- bzw. Zwischenprodukte zur Herstellung von Farbstoffen, Pharmaka und Pflanzenschutzmitteln von Interesse sind oder auch direkt als Pflanzenschutzmittel Verwendung finden (siehe z.B. JP OS 9846/1975 oder Chem. Abstracts 83, 127548 d) durch Umsetzung von Isocyaniddihalogeniden mit Phenolen bzw. Phenolaten erhalten werden.

Ein Nachteil dieser Methode besteht darin, daß die als Edukte verwendeten Isocyaniddihalogenide nur über Umwege aus Aminen herstellbar sind.

Es ist ferner aus J. Heterocycl. Chem. 19, 1205 (1982) bekannt, daß Dichloridiphenoximethan mit Cyanamid zu N-Cyano-iminokohlensäurediphenylester reagiert. N-alkylsubstituierte Iminokohlensäurediphenylester können gemäß der DE-OS 20 33 278 durch Umsetzung von Phenolen mit Dialkylaminotrichlormethan hergestellt werden. Die Dialkylaminotrichlormethanverbindungen, die auch als Imoniumverbindungen aufzufassen sind, sind jedoch nur dann gut zugänglich, wenn die Alkylreste am Stickstoff sterisch nicht sehr anspruchsvoll sind. Vorzugsweise wird die Umsetzung daher mit Dimethylaminotrichlormethan durchgeführt.

Der Erfindung lag die Aufgabe zugrunde, ein einfacheres und wirtschaftliches Verfahren zu finden, nach dem eine Vielzahl verschieden substituierter Iminokohlensäurediarylester zugänglich sind. Durch ein Verfahren, das es gestattet, die Substituenten am Stickstoff und an den Phenoxiresten beliebig zu variieren, sollten Verbindungen mit bislang unbekanntem Substitutionsmuster zur Verfügung gestellt werden.

Demgemäß wurde ein Verfahren zur Herstellung von Iminokohlensäurediarylestern der allgemeinen Formel I

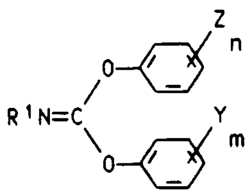

I ,

in der

$R^1$ eine Alkyl-, Aminoalkyl-, Cycloalkyl-, Aralkyl-, Arylgruppe oder einen heterocyclischen oder heteroaromatischen Rest darstellt,

Y und Z gleich oder verschieden sind und eine Alkyl-, Alkoxy-, Halogenalkyl-, Aryl-, Cyano-, Nitrogruppe oder Halogen bedeuten,

m und n für eine ganze Zahl von 1 bis 5 stehen, wobei, wenn m und n größer 1 sind, die Reste Y bzw. Z gleich oder verschieden sind,

gefunden, das dadurch gekennzeichnet ist, daß man Iminokohlensäurediphenylester II

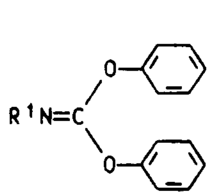

II

mit einem substituierten Phenol III

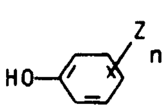

III

bei Temperaturen von 50 bis 250 °C zu Iminokohlensäurediarylestern IV

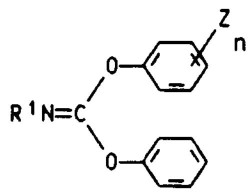

IV

umsetzt und die Verbindungen IV nach deren Isolierung oder in situ mit einem durch Z oder Y substituierten Phenol zu den Endprodukten I umsetzt.

Weiterhin wurde gefunden, daß man Iminokohlensäurediarylester der Formel IV

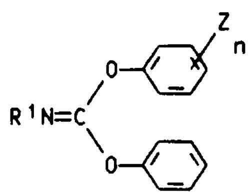

IV,

in der
$R^1$ eine Alkyl-, Aminoalkyl-, Cycloalkyl-, Aralkyl-, Arylgruppe oder einen heterocyclischen oder heteroaromatischen Rest darstellt,
Z eine Alkyl-, Alkoxy-, Halogenalkyl-, Aryl-, Cyano-, Nitrogruppe oder Halogen bedeutet und
n für eine ganze Zahl von 1 bis 5 steht, vorteilhaft erhält, wenn man Iminokohlensäurediphenylester II

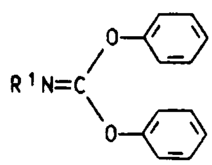

II

mit einem substituierten Phenol III

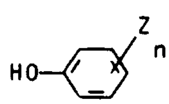

III

bei Temperaturen von 50 bis 150 °C umsetzt, wobei man pro Mol II 1 bis 2 mol III verwendet und dann den Iminokohlensäureester IV in an sich bekannter Weise isoliert.

Weiterhin wurde ein Verfahren zur Herstellung der als Ausgangsstoffe verwendeten Iminokohlensäurediphenylester der allgemeinen Formel II

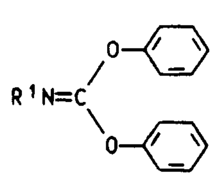

II.

in der $R^1$ eine Alkyl-, Cycloalkyl-, Aralkyl-, Arylgruppe oder einen heterocyclischen oder heteroaromatischen Rest darstellt, gefunden, das dadurch gekennzeichnet ist, daß man Dichlordiphenoximethan mit einem primären Amin $R^1NH_2$ in einem inerten Lösungsmittel in Gegenwart einer Base und gegebenenfalls in Gegenwart einer katalytischen Menge N,N-Dialkylaminopyridin umsetzt und den Iminokohlensäurediphenylester in an sich bekannter Weise isoliert.

Nach den erfindungsgemäßen Verfahren werden Iminokohlensäurediarylester I, II oder IV erhalten, in denen der Substituent am Stickstoff $R^1$ für eine Alkylgruppe, z.B. mit 1 bis 20, insbesondere 1 bis 10 Kohlenstoffatomen, eine Aminoalkylgruppe der Struktur

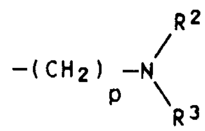 ,

in der
$R^2$ und $R^3$ eine $C_1$- bis $C_8$-, insbesondere $C_1$- bis $C_4$-Alkylgruppe darstellt oder $R^2$ und $R^3$ miteinander verbunden sind und zusammen mit dem Stickstoff einen 5-7gliedrigen Ring, insbesondere einen 5- oder 6-Ring, der noch ein weiteres Heteroatom, wie Schwefel, Stickstoff oder Sauerstoff enthalten kann, bilden und in der der Index p für die Zahlen 1 bis 8, vorzugsweise 1 bis 4, steht. Die Reste $R^2$ und $R^3$ können auch inerte Substituenten, z.B. $C_1$-$C_4$-Alkoxy- oder $C_1$-$C_4$-Alkylreste tragen. Weiterhin steht $R^1$ für eine Cycloalkylgruppe mit 3 bis 8, insbesondere 5 bis 7 Kohlenstoffatomen, eine Aralkylgruppe vorteilhaft mit 7 bis 12 Kohlenstoffatomen, eine Arylgruppe, z.B. eine Phenylgruppe, die unter den Reaktionsbedingungen inerte Substituenten tragen kann, wie Halogenatome, z.B. Fluor, Chlor oder Brom, $C_1$-bis $C_4$-Alkoxy- oder Alkylreste, Halogenalkylreste, insbesondere mit 1 bis 4 Kohlenstoffatomen und 1 bis 3 Halogenatomen, Nitro- oder Cyanogruppen sowie Methyleniminogruppen, z.B. diphenoxysubstituierte Methyleniminogruppen $-N=C(OC_6H_5)_2$ oder Acylaminogruppen

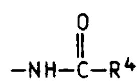 ,

worin $R^4$ eine $C_1$- bis $C_4$-Alkylgruppe bedeutet. Weiterhin steht $R^1$ für heterocyclisches Reste mit 5 bis 8, insbesondere 5 und 6 Ringgliedern, in denen vorzugsweise 1 oder 2 Heteroatome wie Stickstoff, Schwefel oder Sauerstoff vorkommen oder für heterocyclische Reste mit 1 oder 2 der genannten Heteroatome und 5 oder 6 Ringgliedern, denen ein Benzolring ankondensiert sein kann. Auch kann $R^1$ ein heterocyclischer, aromatischer Rest mit 5 oder 6 Ringgliedern und 1 oder 2 der genannten Heteroatome sein, wobei ebenfalls ein Benzolring ankondensiert sein kann.

Als Substituenten $R^1$ am Stickstoff seien beispielhaft folgende Reste aufgeführt: Methyl, Ethyl, Propyl, Isopropyl, Butyl, tert.-Butyl, Hexyl oder Octyl; N,N-Dimethylaminomethyl, N,N-Diethylaminomethyl, N,N-Dimethylaminoethyl,N,N-Dimethylaminopropyl, N,N-Dimethylaminobutyl, 3-N-Pyrrolidylpropyl, 4-N-Morpholinyl-butyl, 3-N-Morpholinylpropyl, 3-N-Piperidylpropyl; Cyclopropyl, Cyclopentyl, Cyclohexyl oder Cyclooctyl; Benzyl-, Phenylethyl-, Phenyl-, Phenyl substituiert durch Fluor, Chlor, Brom, Methoxy, Ethoxy, Methyl, Ethyl, Propyl, Butyl, tert.-Butyl, Dichlormethyl, Trifluormethyl, 2-Chlorethyl, Nitro, Cyano, Diphenoxymethylenimino oder Acetylamino sowie als heterocyclische, bzw. heteroaromatische Reste: Piperidyl-, Pyridyl-, Dihydrofuranyl-, Furanyl-, Chinolinyl-, Pyrazinyl-, Pyrimidyl-, Pyrrolyl-, Benzofuranyl-, Indolyl-, Thiophenyl-, Pyrazolyl-, Imidazolyl-oder Oxazolylreste.

Die Phenylsubstituenten Z bzw. Y sind gleich oder verschieden und stellen eine Alkylgruppe mit 1 bis 9, insbesondere 1 bis 4 Kohlenstoffatomen, z.B. eine Methyl-, Ethyl-, Propyl-, Isopropyl-, Butyl- oder tert.-Butylgruppe, eine $C_1$-$C_4$-Alkoxygruppe, wie Methoxy- oder tert.-Butoxy, eine durch Halogen, z.B. durch Chlor, Brom oder Fluor substituierte Alkylgruppe mit 1 bis 3 Kohlenstoffatomen, z.B. eine Trifluormethyl-, Dichlormethyl- oder 2-Chlorethylgruppe, eine Arylgruppe, insbesondere Phenylgruppe, die durch inerte Gruppen, wie Halogen, $C_1$-$C_4$-Alkyl- oder Alkoxy- oder Nitrogruppen substituiert sein kann, dar. Weiterhin stehen Y bzw. Z für funktionelle Gruppen, wie Nitro- oder Cyanogruppen sowie für Halogen, insbesondere Chlor oder Brom. Vorzugsweise trägt der O-Phenylrest 1 bis 3, insbesondere 1 oder 2 Substituenten Z bzw. Y. Besonders vorteilhaft können nach dem erfindungsgemäßen Verfahren Verbindungen der Formel I, in denen die Bedeutung der Substituenten Y and Z gleich ist, hergestellt werden.

Die erfindungsgemäße Umsetzung des Iminokohlensäurediphenylesters mit dem Phenol III zu I erfolgt in der Weise, daß man mindestens äquimolare Mengen, vorzugsweise einen Überschuß an III, bezogen auf den Ausgangsstoff II, wählt und die Temperatur auf 50 bis 250°C einstellt. Will man das Zwischenprodukt IV isolieren und anschließend mit weiterem Phenol zu I umsetzen, was vorteilhaft sein kann, wenn man Produkte mit unterschiedlichen Substituenten Y und Z herstellen will, so wird man äquimolare Mengen an III oder einen nur geringen Überschuß, z.B. 1 bis 2, insbesondere 1 bis 1,5 mol III pro Mol II verwenden und eine möglichst geringe Reaktionstemperatur, z.B. eine Temperatur von 50 bis 150°C wählen und erst im zweiten Reaktionsschritt, der Umsetzung mit einem durch Y substituierten Phenol, die Reaktionstempertur auf bis zu 250°C, vorteilhaft 100 bis 250°C, erhöhen. Die Menge des durch Y substituierten Phenols kann 1 bis 3 mol, insbesondere 1 bis 2 mol pro Mol des Zwischenprodukts IV betragen. Der Austausch der Phenylgruppen kann in Gegenwart oder Abwesenheit von Lösungsmitteln erfolgen. Geeignete inerte Lösungsmittel sind beispielsweise hochsiedende Kohlenwasserstoffe, halogenierte Kohlenwasserstoffe, wie Chlorbenzol, Brombenzol oder Dichlorbenzol oder Glykolether.

Nach einer bevorzugten Ausführungsform des Verfahrens werden Iminokohlensäurediarylester I mit identischen O-Arylresten hergestellt. In diesem Fall bringt eine Isolierung des Zwischenprodukts IV im allgemeinen keine Vorteile. Vorzugsweise kann man einen Überschuß des phenolischen Reaktanten III, z.B. 2,1 bis 20, insbesondere 2,5 bis 10 mol pro Mol II, verwenden und die Reaktion in Abwesenheit eines Lösungsmittels durchführen. Die Reaktionstemperatur wird so hoch gehalten, daß der Phenolaustausch vollständig stattfindet. Im allgemeinen sind Temperaturen von 50 bis 250°C, insbesondere 70 bis 200, vorzugsweise 80 bis 170°C ausreichend. Es ist auch möglich, verschiedene Temperaturbereiche zu wählen und die Umsetzung zunächst bei niedrigerer Temperatur von ca. 50 bis 100°C zu beginnen und dann die Temperatur auf ca. 100 bis 200 oder 150 bis 250°C zu erhöhen.

Das im Überschuß eingesetzte substituierte Phenol kann vorteilhaft zurückgewonnen und für weitere Umsetzungen wieder verwendet werden, indem man es z.B. destillativ aus dem Reaktionsgemisch abtrennt oder das Reaktionsgemisch wässrig aufarbeitet, indem man ein inertes organisches Lösungsmittel, z.B. einen Ether oder aliphatischen Kohlenwasserstoff hinzufügt und das Phenol mit Wasser, dem gegebenenfalls eine Base, wie ein Alkalicarbonat hinzugefügt wird, extrahiert.

Auch das bei der Umsetzung freigesetzte Phenol läßt sich in an sich bekannter Weise abtrennen, z.B. indem man die Reaktion nicht bei Normaldruck, sondern einem geringen Unterdruck durchführt.

Die Isolierung und Reinigung der Iminokohlensäurediarylester I bzw. IV aus dem Umsetzungsgemisch kann in an sich bekannter Weise, z.B. durch destillative Abtrennung der flüchtigeren Komponenten und ggf. Umkristallisieren der Produkte erfolgen, so daß sich weitere Ausführungen hierzu erübrigen.

Die als Ausgangsstoffe für die oben beschriebene Phenolaustauschreaktion verwendeten Iminokohlensäurediphenylester werden erfindungsgemäß durch Umsetzung von Dichlordiphenoximethan, das z.B. wie in J. Heterocycl. Chem. 19, 1205 (1982) beschrieben, erhalten werden kann, mit einem primären Amin $R^1NH_2$ in Gegenwart einer Base gemäß folgender Reaktionsgleichung hergestellt:

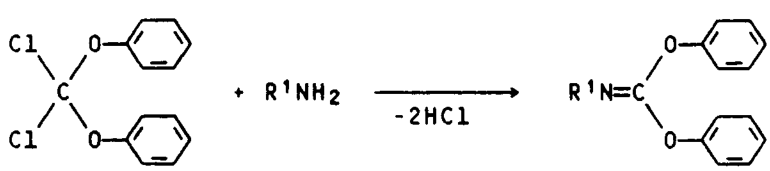


Pro Mol Dichlordiphenoximethan können vorteilhaft 1 bis 1,5, insbesondere 1 bis 1,2 mol des primären Amins verwendet werden. Bevorzugt werden äquimolare Mengen beider Reaktanten umgesetzt.

Die Reaktion erfolgt in Gegenwart einer Base, die geeignet ist, den entstehenden Chlorwasserstoff zu binden. Beispielsweise seien als Basen tertiäre Amine genannt, wobei Trimethylamin, Triethylamin, Tributylamin oder Pyridin besonders vorteilhaft sind. Die Menge der Base ist nicht besonders kritisch, vorzugsweise kann man 2 bis 2,5 mol pro Mol Dichlordiphenoximethan verwenden. Höhere Mengen sind möglich, bringen aber keine Vorteile.

Zur Beschleunigung der Umsetzung kann es häufig vorteilhaft sein, dem Umsetzungsgemisch katalytische Mengen an N,N-Dialkylaminopyridin, z.B. ca. $10^{-3}$ bis $10^{-2}$ mol pro Mol Dichlordiphenoximethan zuzusetzen. Dialkylaminopyridine sind z.B. 4-Dimethylamino-, 3-Diethylamino- oder 4-Dibutylaminopyridin.

Die Umsetzung wird in Gegenwart eines inerten organischen Lösungsmittels durchgeführt. Geeignete Lösungsmittel sind z.B. aliphatische oder cycloaliphatische Kohlenwasserstoffe, wie Pentan, Hexan oder

Cyclohexan, Ether, wie Diethylether, Tetrahydrofuran, Dioxan oder halogenierte aliphatische oder cycloaliphatische Kohlenwasserstoffe, wie Methylenchlorid oder Chloroform sowie Aromaten, wie Benzol oder Toluol. Die Lösungsmittelmenge beträgt im allgemeinen 0,5 bis 1 l pro Mol Dichlordiphenoximethan.

Die Reaktionstemperatur kann sehr stark variiert werden. So sind Temperaturen von 0 bis 150, insbesondere 10 bis 100 °C möglich. Bevorzugt wird man die Reaktion bei Temperaturen von 0 bis 25 °C durchführen.

Nach erfolgter Umsetzung kann das Reaktionsgemisch in an sich üblicher Weise aufgearbeitet werden, z.B. indem man das gebildete Ammoniumsalz abfiltriert und das Lösungsmittel abtrennt. Die verbleibenden festen Produkte sind im allgemeinen so rein, daß sich ein anschließendes Umkristallisieren erübrigt.

Gemäß dem erfindungsgemäßen Verfahren können letztlich ausgehend von primären Aminen, deren Substituenten breit variierbar sind, auf einfachem Wege und in hohen Ausbeuten Iminokohlensäurediarylester gewonnen werden, deren Aryloxyreste praktisch beliebig substituiert sein können.

Für die Weiterverwendung besonders interessante Verbindungen sind Iminokohlensäurediphenylester der Formel II,

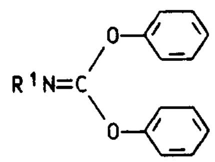

II,

in der $R^1$ eine tert.-Alkylgruppe, eine Aminoalkylgruppe, einen Cycloalkylrest, einen heterocyclischen Rest mit 5 bis 8 Ringgliedern und 1 oder 2 Heteroatomen, einen heteroaromatischen Rest mit 1 oder 2 Heteroatomen oder Phenyl, substituiert durch $C_1$-$C_4$-Alkoxy-, Halogenalkyl-, Acylamino- oder Methyleniminogruppen bedeutet sowie Iminokohlensäurediarylester der Formel V

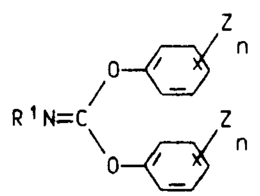

V,

in der $R^1$ die in Anspruch 10 genannte Bedeutung hat, Z für Halogen, $C_1$-$C_4$-Alkyl, $C_1$-$C_4$-Alkoxy oder Phenyl steht und n die Zahl 1 oder 2 darstellt.

Tertiäre Alkylreste sind z.B. solche mit 4 bis 10, insbesondere 4 bis 8 Kohlenstoffatomen, wie tert.-Butyl oder tert.-Pentyl. Aminoalkylgruppen sind solche der oben definierten Struktur

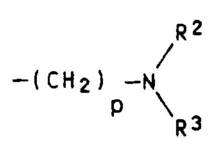

,

z.B. N,N-Dimethylaminomethyl, N,N-Diethylaminomethyl, N,N-Dimethylaminoethyl, N,N-Dimethylaminopropyl, N,N-Dimethylaminobutyl, 3-N-Pyrrolidylpropyl, 4-N-Morpholinyl-butyl, 3-N-Morpholinylpropyl, 3-N-Piperidylpropyl; Cycloalkylreste sind $C_5$- bis $C_8$-Cycloalkylreste, insbesondere Cyclopentyl oder Cyclohexyl. Heterocyclische Reste oder heteroaromatische Reste sind die oben definierten Reste, insbesondere solche mit 5 oder 6 Ringgliedern und 1 oder 2 Heteroatomen wie Schwefel, Sauerstoff oder Stickstoff, denen ein Benzolrest ankondensiert sein kann, beispielsweise Piperidyl-, 4-(2,2,6,6-Tetramethyl)-piperidyl-, Pyridyl-, Dihydrofuranyl-, Furanyl-, Chinolinyl-, Pyrazinyl-, Pyrimidyl-, Pyrrolyl-, Benzofuranyl-, Indolyl-, Thiophenyl-, Pyrazolyl-, Imidazolyl-oder Oxazolylreste. Substituenten am Phenylrest sind z.B. $C_1$- bis $C_4$-Alkoxy, wie Methoxy, Ethoxy, Propoxy, tert. Butoxy, Halogenalkyl, insbesondere mit 1 bis 4 Kohlenstoffatomen und 1

bis 3 Halogenatomen, z.B. Trifluormethyl, Dichlormethyl, Dibrommethyl, 2-Chlorethyl oder 1,2,2-Trichlorethyl. Acylaminoreste haben z.B. die Struktur

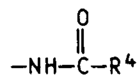
,

worin $R^4$ eine $C_1$- bis $C_4$-Alkylgruppe bedeutet, wobei die Acetylgruppe bevorzugt ist. In Methyleniminogruppen $-N=CR^5{}_2$, steht $R^5$ vorzugsweise für Phenoxireste.

Die erfindungsgemäßen Verbindungen sind wie eingangs erwähnt wichtige Zwischenprodukte, insbesondere können sie z.B. wie in Chem. Ber. 117, Seite 2579, 1984, Journal of Heterocyclic Chemistry 21, 753-757, 1984 oder in US-PS 4 285 878 beschrieben zu Isoharnstoffen weiterverarbeitet werden.

Das erfindungsgemäße Verfahren sei an folgenden Beispielen erläutert:

Beispiele 1 bis 7:

Herstellung von Verbindungen der allgemeinen Formel I

17,5 g (50 mmol) N-(2,5-dimethoxy-phenyl)-iminokohlensäurediphenylester wurden mit 25,7 g (200 mmol) 4-Chlorphenol durchmischt und unter Rühren und Anlegen eines schwachen Vakuums (Wasserstrahlpumpe) auf 150 ° C erhitzt. Nach 2 Stunden wurde alles überschüssige Phenol abdestilliert und man erhielt 19,3 g (92%) N-(2,5-dimethoxy-phenyl)iminokohlensäure-di-(4-chlorphenyl)-ester.

| Elementaranalyse: | | | | |
|---|---|---|---|---|
| berechnet: | C 60,3 | H 4,1 | N 3,3 | Cl 16,9 |
| gefunden: | C 60,6 | H 4.3 | N 3,3 | Cl 16,7 |

Analog wurden die in Tabelle 1 aufgeführten Verbindungen der Formel I

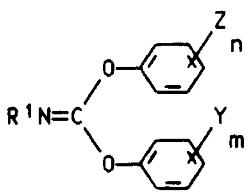
I

hergestellt, wobei die Aryloxireste identisch waren.

Tabelle 1

| Bsp. | $R^1$ | 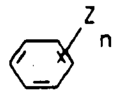 | Ausb. | Smp. | Elementaranalyse | | | | |
|---|---|---|---|---|---|---|---|---|---|
| | | | % | °C | C | H | N | Cl | F |
| 2 | Cyclopropyl | 4-Chlorphenyl | 74 | 72 | ber.59.6<br>gef.59.5 | 4.1<br>4.2 | 4.3<br>4.3 | 22.0<br>21.0 | |
| 3 | Cyclopropyl | p-Biphenyl | 60 | 126-<br>127 | ber.82.9<br>gef.82.4 | 5.7<br>5.6 | 3.4<br>3.0 | | |
| 4 | Cyclopentyl | 4-Chlorphenyl | 94 | 43-<br>44.5 | ber.61.7<br>gef.61.7 | 4.9<br>4.9 | 4.0<br>3.9 | 20.2<br>20.1 | |
| 5 | 3-Trifluor-<br>methylphenyl | 4-Chlorphenyl | 86 | 65.5<br>-67 | ber.56.3<br>gef.56.3 | 2.8<br>3.0 | 3.3<br>3.3 | 16.6<br>16.4 | 13.3<br>13.5 |
| 6 | Cyclohexyl | 4-Chlorphenyl | 92 | Öl | ber.62.6<br>gef.62.6 | 5.3<br>5.3 | 3.8<br>4.1 | 19.4<br>19.0 | |
| 7 | Cycloheptyl | 4-Chlorphenyl | 79 | Öl | ber.63.5<br>gef.63.5 | 5.6<br>5.7 | 3.7<br>3.8 | 18.7<br>17.9 | |
| 8 | Cyclopropyl | 2.4-Dichlor-<br>phenyl | 81 | Öl | ber.49.2<br>gef.49.8 | 2.8<br>3.0 | 3.6<br>3.6 | 36.2<br>35.0 | |
| 9 | Cyclopropyl | 4-Methoxy-<br>phenyl | 86 | Öl | ber.68.9<br>gef.68.3 | 6.1<br>6.4 | 4.5<br>5.1 | | |

Beispiele 10 - 22

Herstellung von Verbindungen der allgemeinen Formel II

8,07 g (30 mmol) Dichlordiphenoxymethan wurden mit 6,66 g (66 mmol) Triethylamin in 200 ml THF gelöst. Nach Zugabe einer Spatelspitze Dimethylaminopyridin wurden 3,83 g (30 mmol) 4-Chloranilin, gelöst in 20 ml THF, langsam zugetropft. Man ließ 24 Stunden rühren und filtrierte das Ammoniumsalz ab. Nach Entfernen des Lösungsmittels wurden 9,6 g (99%) N-(4-Chlorphenyl)-iminokohlensäurediphenylester vom Smp. 96 °C erhalten.

Analog wurden die in Tabelle 2 aufgeführten Verbindungen der Formel

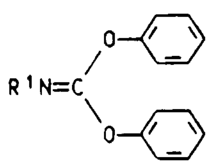

II

hergestellt.

Tabelle 2

| Bsp. | $R^1$ | Ausb. % | Smp. °C | Elementaranalyse C | H | N | Cl |
|---|---|---|---|---|---|---|---|
| 10 | Cyclopropyl | 97 | 62 | ber. 75.8<br>gef. 75.6 | 6.0<br>6.4 | 5.5<br>5.6 | |
| 11 | Cyclopentyl | 95 | 51-53 | ber. 76.8<br>gef. 76.2 | 6.8<br>6.8 | 5.0<br>5.2 | |
| 12 | 3-Trifluormethylphenyl | 96 | Öl | | - | | |
| 13 | 3-Pyridyl | 93 | 64-66 | ber. 74.4<br>gef. 73.3 | 4.9<br>5.0 | 9.7<br>9.7 | |
| 14 | 2,5-Dimethoxyphenyl | 93 | 107 | ber. 72.2<br>gef. 72.2 | 5.5<br>5.5 | 4.0<br>3.9 | |
| 15 | 4-Acetaminophenyl | 90 | 164 | ber. 72.8<br>gef. 72.6 | 5.2<br>5.4 | 8.0<br>8.1 | |
| 16 | 2-Pyridyl | 85 | 115 | ber. 74.4<br>gef. 73.9 | 4.9<br>4.9 | 9.7<br>10.2 | |
| 17 | 3-Dimethylaminopropyl | 87 | Öl | ber. 71.9<br>gef. 72.3 | 8.0<br>7.6 | 9.3<br>9.3 | |
| 18 | t-Butyl | 94 | 61-62 | ber. 75.8<br>gef. 75.4 | 7.1<br>7.2 | 5.2<br>5.1 | |
| 19 | 2-(4-Chlorphenyl)ethyl | 83 | 57-58 | ber. 71.6<br>gef. 70.7 | 5.1<br>5.6 | 4.0<br>4.0 | 10.1<br>9.5 |
| 20 | 4-(2,2'-Diphenoxymethyleniminophenyl | 86 | 201<br>204 | ber. 76.7<br>gef. 76.1 | 4.8<br>5.0 | 5.7<br>5.7 | |
| 21 | 3-N-Morpholinylpropyl | 92 | Öl | ber. 70.5<br>gef. 70.1 | 7.1<br>6.6 | 8.2<br>7.8 | |
| 22 | 4-(2.2.6.6-Tetramethyl)piperidinyl | 86 | Öl | ber. 74.9<br>gef. 75.4 | 8.0<br>7.1 | 7.9<br>7.4 | |
| 23 | 3-N-(2'-Methylpiperidinyl)propyl | 92 | Öl | ber. 74.9<br>gef. 74.6 | 8.0<br>7.8 | 7.9<br>8.2 | |

## Patentansprüche

**1.** Verfahren zur Herstellung von Iminokohlensäurediarylestern der allgemeinen Formel I

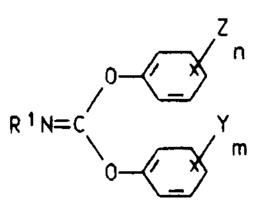

I,

in der
$R^1$ eine Alkyl-, Aminoalkyl-, Cycloalkyl-, Aralkyl-, Arylgruppe oder einen heterocyclischen oder heteroaromatischen Rest darstellt,
Y und Z gleich oder verschieden sind und eine Alkyl-, Alkoxy-, Halogenalkyl-, Aryl-, Cyano-, Nitrogruppe oder Halogen bedeuten,
m und n für eine ganze Zahl von 1 bis 5 stehen, wobei, wenn m und n größer 1 sind, die Reste Y bzw.

Z gleich oder verschieden sind,
dadurch gekennzeichnet, daß man Iminokohlensäurediphenylester II

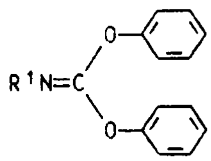

II

mit einem substituierten Phenol III

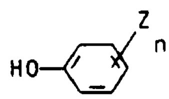

III

bei Temperaturen von 50 bis 250 ° C zu Iminokohlensäurediarylestern IV

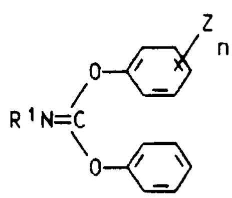

IV

umsetzt und die Verbindungen IV nach deren Isolierung oder in situ mit einem durch Z oder Y substituierten Phenol zu den Endprodukten I umsetzt.

**2.** Verfahren nach Anspruch 1, dadurch gekennzeichnet, daß man 2,1 bis 20 mol Phenol III je Mol Iminokohlensäurediphenylester II direkt zum Diarylester der Formel I umsetzt, in der $Y_m$ und $Z_n$ die gleiche Bedeutung haben.

**3.** Verfahren nach Anspruch 1, dadurch gekennzeichnet, daß man zunächst bei Temperaturen von 50 bis 150 ° C den Iminokohlensäurediarylester IV herstellt, wobei man pro Mol Diphenylester II 1 bis 2 mol Phenol III verwendet und dann je Mol Diarylester IV mit 1 bis 3 Mol Y substituiertem Phenol bei Temperaturen von 100 bis 250 ° C zum Endprodukt I umsetzt.

**4.** Verfahren nach den Ansprüchen 1 bis 3, dadurch gekennzeichnet, daß man im Überschuß verwendetes Phenol III zurückgewinnt und für weitere Umsetzungen verwendet.

**5.** Verfahren zur Herstellung von Iminokohlensäurediarylestern der Formel IV

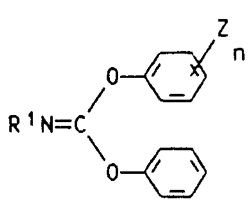

IV,

in der
$R^1$ eine Alkyl-, Aminoalkyl-, Cycloalkyl-, Aralkyl-, Arylgruppe oder einen heterocyclischen oder heteroaromatischen Rest darstellt,

Z eine Alkyl-, Alkyloxy-, Halogenalkyl-, Aryl-, Cyano-, Nitrogruppe oder Halogen bedeutet und
n für eine ganze Zahl von 1 bis 5 steht dadurch gekennzeichnet, daß man Iminokohlensäurediphenylester II

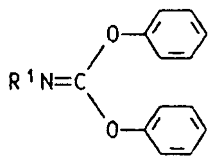

II

mit einem substituierten Phenol III

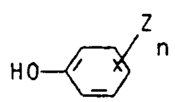

III

bei Temperaturen von 50 bis 150 °C umsetzt, wobei man pro Mol II 1 bis 2 mol III verwendet und dann den Iminokohlensäureester IV in an sich bekannter Weise isoliert.

**6.** Verfahren zur Herstellung von Iminokohlensäurediphenylestern der allgemeinen Formel II

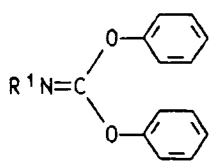

II,

in der $R^1$ eine Alkyl-, Cycloalkyl-, Arylgruppe oder einen heterocyclischen oder heteroaromatischen Rest darstellt, dadurch gekennzeichnet, daß man Dichlordiphenoximethan mit einem primären Amin $R^1NH_2$ in einem inerten organischen Lösungsmittel in Gegenwart einer Base und gegebenenfalls in Gegenwart einer katalytischen Menge N,N-Dialkylaminopyridin umsetzt und den Iminokohlensäurediphenylester in an sich bekannter Weise isoliert.

**7.** Verfahren nach Anspruch 6, dadurch gekennzeichnet, daß man pro Mol Dichlordiphenoximethan 1 bis 1,5 mol Amin verwendet.

**8.** Verfahren nach Anspruch 6, dadurch gekennzeichnet, daß man als Base ein tertiäres Amin verwendet.

**9.** Iminokohlensäurediphenylester der Formel II,

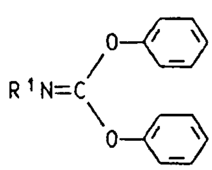

II,

in der $R^1$ einen tert.-Alkylrest, einen Aminoalkylrest, einen Cycloalkylrest, einen heterocyclischen Rest mit 5 bis 8 Ringgliedern und 1 oder 2 Heteroatomen, einen heteroaromatischen Rest mit 1 oder 2 Heteroatomen oder Phenyl, substituiert durch $C_1$-$C_4$-Alkoxy-, Halogenalkyl-, Acylamino- oder Methyleniminogruppen bedeutet.

**10.** Iminokohlensäurediarylester der Formel V

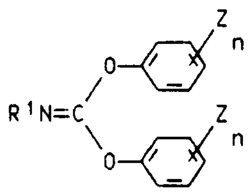

V,

in der
$R^1$ die in Anspruch 9 genannte Bedeutung hat,
Z für Halogen, $C_1$-$C_4$-Alkyl, $C_1$-$C_4$-Alkoxy oder Phenyl steht und
n die Zahl 1 oder 2 darstellt.

**Claims**

**1.** A process for preparing diaryl iminocarbonates of the formula I

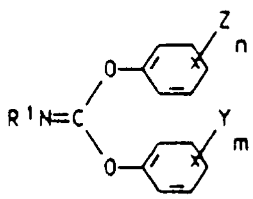

I

where
$R^1$ is alkyl, aminoalkyl, cycloalkyl, aralkyl or aryl or a heterocyclic or heteroaromatic radical,
Y and Z are identical or different and are alkyl, alkyloxy, haloalkyl, aryl, cyano, nitro or halogen,
m and n are an integer from 1 to 5, where the radicals Y and Z are identical or different when m and n are greater than 1, which comprises reacting diphenyl iminocarbonates II

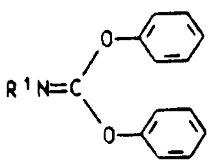

II

with a substituted phenol III

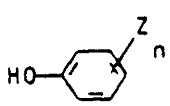

III

at from 50 to 250 °C to give diaryl iminocarbonates IV

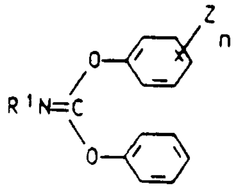

IV

and reacting the compounds IV, after the isolation thereof or in situ, with a phenol substituted by Z or Y

to give the final products I.

2. A process as claimed in claim 1, wherein from 2.1 to 20 mol of phenol III are reacted per mole of diphenyl iminocarbonate II directly to the diaryl ester of the formula I where $Y_m$ and $Z_n$ have the same meaning.

3. A process as claimed in claim 1, wherein the diaryl iminocarbonate IV is initially prepared at from 50 to 150 °C, using from 1 to 2 mol of phenol III per mole of diphenylester II, and then from 1 to 3 mole of Y-substituted phenol are reacted per mole of diaryl ester IV at from 100 to 250 °C to give the final product I.

4. A process as claimed in claims 1 to 3, wherein phenol III used in excess is recovered and used for further reactions.

5. A process for preparing diaryl iminocarbonates of the formula IV

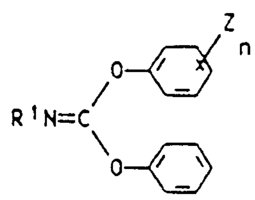

IV

where
$R^1$ is alkyl, aminoalkyl, cycloalkyl, aralkyl or aryl or a heterocyclic or heteroaromatic radical,
Z is alkyl, alkyloxy, haloalkyl, aryl, cyano, nitro or halogen, and
n is an integer from 1 to 5, which comprises reacting diphenyl iminocarbonates II

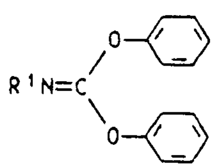

II

with a substituted phenol III

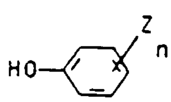

III

at from 50 to 150 °C, using from 1 to 2 mol of III per mole of II, and then isolating the iminocarbonic ester IV in a conventional manner.

6. A process for preparing diphenyl iminocarbonates of the formula II

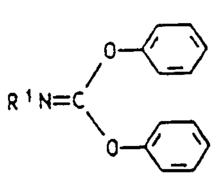

II

where $R^1$ is alkyl, cycloalkyl or aryl or a heterocyclic or heteroaromatic radical, which comprises

reacting dichlorodiphenoxymethane with a primary amine $R^1NH_2$ in an inert organic solvent in the presence of a base and in the presence or absence of a catalytic amount of N,N-dialkylaminopyridine, and isolating the diphenyl iminocarbonate in a conventional manner.

**7.** A process as claimed in claim 6, wherein from 1 to 1.5 mol of amine is used per mole of dichlorodiphenoxymethane.

**8.** A process as claimed in claim 6, wherein a tertiary amine is used as base.

**9.** A diphenyl iminocarbonate of the formula II

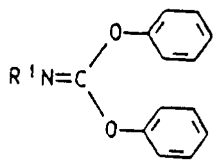

II

where $R^1$ is tert-alkyl, aminoalkyl, cycloalkyl, a heterocyclic radical with from 5 to 8 ring members and 1 or 2 hetero atoms, a heteroaromatic radical with from 1 to 2 hetero atoms or phenyl substituted by $C_1$-$C_4$-alkoxy, haloalkyl, acylamino or methyleneimino groups.

**10.** A diaryl iminocarbonate of the formula V

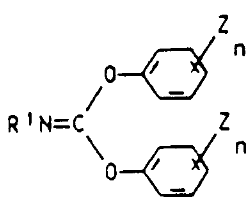

V

where $R^1$ has the meaning specified in claim 9,
Z is halogen, $C_1$-$C_4$-alkyl, $C_1$-$C_4$-alkoxy or phenyl, and
n is the number 1 or 2.

**Revendications**

**1.** Procédé de préparation d'iminocarbonates de diaryle de formule générale I

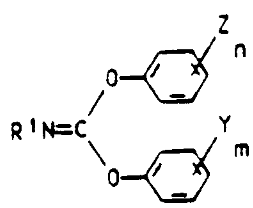

I,

dans laquelle

- $R^1$ représente un groupement alkyle, aminoalkyle, cycloalkyle, aralkyle ou aryle ou un reste hétérocyclique ou hétéroaromatique,
- Y et Z sont identiques ou différents et représentent chacun un groupement alkyle, alkyloxy, halogénoalkyle, aryle, cyano ou nitro ou un atome d'halogène,
- m et n sont mis chacun pour un nombre entier de 1 à 5, les restes Y ou Z étant identiques ou différents lorsque m et n sont supérieurs à 1,

caractérisé en ce qu'on fait réagir des iminocarbonates de diphényle II

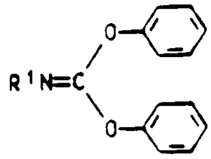

II

avec un phénol substitué III

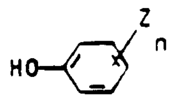

III

à des températures de 50 à 250 ° C pour obtenir des iminocarbonates de diaryle IV

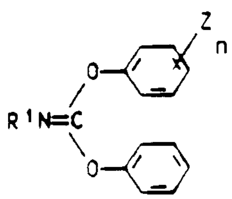

IV

et on fait réagir les composés IV, après les avoir isolés ou in situ, avec un phénol substitué par Z ou Y, pour obtenir les produits finals I.

**2.** Procédé selon la revendication 1, caractérisé en ce qu'on fait réagir de 2,1 à 20 moles de phénol III par mole d'iminocarbonate de diphényle II pour obtenir directement l'ester diarylique de formule I, dans laquelle $Y_m$ et $Z_n$ ont la même signification.

**3.** Procédé selon la revendication 1, caractérisé en ce qu'on commence par préparer l'iminocarbonate de diaryle IV à des températures de 50 à 150 ° C, en utilisant 1 à 2 moles de phénol III par mole d'ester diphénylique, puis on fait réagir 1 à 3 modes de phénol substitué par Y par mole d'ester diarylique IV à des températures de 100 à 250 ° C, pour obtenir le produit final I.

**4.** Procédé selon l'une quelconque des revendications 1 à 3, caractérisé en ce qu'on récupère le phénol III utilisé en excès et on l'utilise pour des réactions ultérieures.

**5.** Procédé de préparation d'iminocarbonates de diaryle de formule générale IV

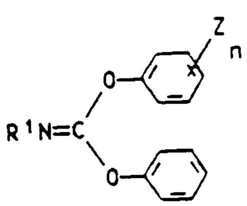

IV.

dans laquelle

$R^1$ représente un groupement alkyle, aminoalkyle, cycloalkyle, aralkyle ou aryle ou un reste hétérocyclique ou hétéroaromatique,

Z représente un groupement alkyle, alkyloxy, halogénoalkyle, aryle, cyano ou nitro ou un atome d'halogène,

n est mis pour un nombre entier de 1 à 5,

caractérisé en ce qu'on fait réagir des iminocarbonatesde diphényle II

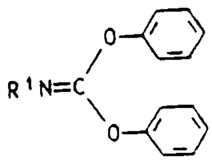

II

avec un phénol substitué III

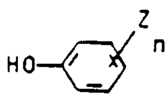

III

à des températures de 50 à 150°C, en utilisant 1 à 2 modes de III par mode de II, puis on isole l'iminocarbonate IV de façon en soi connue.

**6.** Procédé de préparation d'iminocarbonates de diphényle de formule générale II

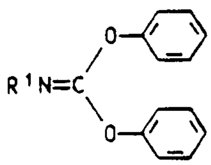

II.

dans laquelle

$R^1$ représente un groupement alkyle, cycloalkyle ou aryle ou un reste hétérocyclique ou hétéroaromatique,

caractérisé en ce qu'on fait réagir du dichlorodiphénoxyméthane avec une amine primaire $R^1NH_2$ dans un solvant organique inerte en présence d'une base et éventuellement en présence d'une quantité catalytique de N,N-dialkylaminopyridine et on isole l'iminocarbonate de diphényle de façon connue en soi.

**7.** Procédé selon la revendication 6, caractérisé en ce qu'on utilise de 1 à 1,5 mode d'amine par mole de dichlorodiphénoxyméthane.

**8.** Procédé selon la revendication 6, caractérisé en ce qu'on utilise, comme base, une amine tertiaire.

**9.** Iminocarbonates de diphényle de formule II

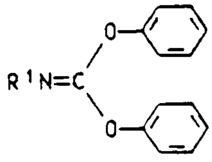

II.

dans laquelle $R^1$ représente un reste tert.-alkyle, un reste aminoalkyle, un reste cycloalkyle, un reste hétérocyclique à 5-8 termes cycliques et 1 ou 2 hétéroatomes, un reste hétéroaromatique à 1 ou 2 htéroatomes ou un reste phényle substitué par des groupements alcoxy en $C_1$ à $C_4$, halogénoalkyle, acylamino ou méthylène-imino.

**10.** Iminocarbonates de diaryle de fomule V

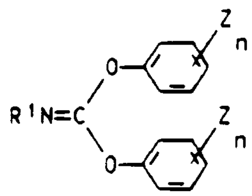

V,

dans laquelle

$R^1$ a la signification donnée dans la revendication 9,

Z est mis pour un atome d'halogène ou pour un reste alkyle en $C_1$-$C_4$, alcoxy en $C_1$-$C_4$ ou phényle et

n représente le nombre 1 ou 2.